# FASCICULE DE BREVET EUROPEEN

(11) **EP 1 085 081 B1**
(45) Date de publication et mention de la délivrance du brevet: **15.04.2009**
(21) Numéro de dépôt: 00402555.7
(22) Date de dépôt: 15.09.2000
(51) Int. Cl.: C12M 3/04, B65D 75/30, B65D 81/20, B65D 81/22, B65D 85/50, A01N 1/02, A61F 15/00, C12N 5/06

(54) **Procédé pour le transport des cellules et des greffes**
Verfahren zum Transport von Zellen und Transplantaten
Method for the transport of cells and grafts

(30) Priorité: 17.09.1999 FR 9911632
(43) Date de publication de la demande: 21.03.2001
(73) Titulaire: Laboratoires Genevrier, 06600 Antibes (FR)
(72) Inventeur: Vacher, Dominique, Domaine de la Peyrière 05250 Mougins (FR); Biehlman, Florence, 06600 Antibes (FR); Guillot, François, 06600 Antibes (FR)
(74) Mandataire: Célanie, Christian

(56) Documents cités:
- EP-A- 0 735 135
- WO-A-93/08776
- US-A- 5 040 677
- US-A- 5 100 676
- US-A- 5 593 880

## Description

La présente invention se rapporte au domaine de la biochimie et en particulier au domaine de la culture des cellules.

Elle a plus précisément pour objet un procédé de mise en culture de cellules humaines et leur transport, sans endommager le potentiel de multiplication des cellules au cours de ce transport.

Elle a spécifiquement pour objet un moyen permettant d'assurer d'une manière optimale le transport en cours de culture, de cellules humaines et notamment de kératinocytes provenant de fragments d'épiderme, caractérisé en ce que :
- les feuillets d'épiderme sont placés sur un milieu de culture semi-solide spécifiquement développé à base d'un hydrogel aqueux formé d'un polymère naturel ou synthétique ou d'un polymère hémi-synthétique ou d'une protéine naturelle ou modifiée. Aucun milieu liquide n'est en effet agencé pour éviter un frottement entre les cultures basales et par conséquent éviter tout dommage,
- les épidermes ou épithéliums en culture sont pincés à l'aide d'un clip (« clipsés ») à un pansement non adhérent inoffensif,
- les feuillets d'épiderme sont emballés sous flux laminaire dans un blister stérile double, étanche à l'eau et dans des conditions favorables à l'utilisateur,
- le blister stérile intérieur est soumis à une injection de gaz contenant du CO₂ pour stimuler la prolifération des cellules épidermiques, et notamment des kératinocytes,
- les blisters stériles sous pression de gaz carbonique sont conditionnés à l'intérieur d'une boîte isothermique pour le transport avec des « nodules » spécifiques qui peuvent absorber et/ou engendrer de la chaleur par transport d'énergie. De cette façon, la température pendant le transport est ajustée à 18-28°C pour éviter la quiescence des kératinocytes et les feuillets d'épiderme sont éventuellement transférés dans un milieu de transport.

De nombreuses publications ont démontré que de grandes quantités d'épithélium, après mise en culture, peuvent être engendrées à partir d'un petit morceau d'épiderme et qu'elles peuvent être employées pour des applications en thérapie cellulaire. Lorsque les kératinocytes sont mis en culture non loin du théâtre d'opération, et même de préférence à proximité, la délivrance de greffe ne pose que des problèmes mineurs. Au contraire, lorsqu'on effectue la culture d'épithélium en un endroit, dans la perspective d'assurer une possibilité de service à l'extérieur, à plus ou moins grande distance, le transport devient un obstacle sérieux et la qualité des cellules peut être gravement mise en danger par le transport.

La viabilité des cellules après transport (avant le greffage de feuillets d'épithélium autologues selon Rheinwald et Green), a été étudiée en se basant sur l'efficacité des kératinocytes à former des colonies. Les unités formant des colonies (CFU), les zones de colonies et les nombres de kératinocytes ont été analysés. En outre, on a étudié l'indice mitotique des kératinocytes (en employant un anticorps contre l'antigène Ki-67), la présence d'intégrines spécifiques (α6β4 et α3β1), les kératines 14 et 10 et l'antigène du pemphigus bulleux (BPA) de 180 Kda à l'intérieur de la feuille d'épiderme après transport. Les résultats ont été comparés aux données provenant d'épithélium mis en culture, similaires juste après détachement par la dispase 24 heures plus tôt.

Les paramètres pour le transport optimal sont décrits ci-dessous :

Après rinçage du feuillet épidermique « clipsé » au pansement non adhérent par un tampon type PBS dans le poste de sécurité microbiologique à flux laminaire, l'opérateur saisit le plateau supérieur (ou petite barquette), contenant le milieu de transport semi-solide et le sort de sa pochette stérile, puis le transfère dans la petite empreinte de scellement, à l'aide de pinces stériles.

A l'aide de deux longues pinces stériles, l'opérateur transfère le greffon de sa boîte de culture et le met en place, dans le plateau supérieur. Les cellules sont alors mises en contact direct avec le milieu semi-solide.

Enfin, l'opérateur positionne une portion de film pelable et découpé aux dimensions appropriées, s'assure de son sens et de son orientation, et le dispose à l'emplacement prévu sur la petite barquette.
La barquette contient un milieu de culture semi-solide constitué d'un hydrogel déshydratable (ou non) qui peut être formé d'un milieu de culture et d'une matière première naturelle réactive comme la carrhagénine, la gomme xanthane, les alginates, l'agarose, les celluloses modifiées chimiquement, le gluten, les albumines végétales, ou de matières premières semi-synthétiques comme la farine de pois ou la caséine condensée à un agent réticulant comme un ester réactif de polyéthylèneglycol ou un éther bisglycidique, ou bien encore de la farine de blé réticulée par du formol ou de l'aldéhyde glutarique ou bien encore de l'acide hyaluronique réticulé ou inter-réticulé ; la matière première pourra aussi être un polymère synthétique comme l'acide acrylique, l'acide méthacrylique ou un acrylamide éventuellement substitué à l'azote.

On distingue sous le nom de « barquettes » ou de blisters doubles, étanches à l'eau, un type particulier de boîtages coulissants destinés à recevoir et à emballer des fragments de tissu cultivés en milieu stérile.

Concrètement, lorsque le milieu de transport semi-solide est préparé, il est coulé dans la petite barquette et il faut attendre qu'il devienne solide. On place alors la petite barquette non scellée dans la grande barquette et on scelle ensuite l'ensemble.

Le substrat de culture utilisé pour le transport peut être un milieu semi-solide formé d'un polymère naturel ou synthétique, comme par exemple un gel de chitine ayant un degré d'acétylation d'au moins 60 % du milieu de culture cellulaire comme agent dispersant ou un gel de chitosanne formant ainsi un réseau tridimensionnel dont la structure est fonction notamment des conditions expérimentales de formation du gel.

Le gel de chitine plus ou moins acétylée est incolore, transparent et sensiblement rigide tout en conservant une certaine aptitude à la déformation en fonction de son épaisseur. Il est souhaitable que la quantité de chitine en poids par rapport au gel soit comprise entre 0,5 et 3,5 %.

Le substrat de culture peut être également un milieu semi-solide sous forme d'hydrogel contenant 95 % de milieu de culture cellulaire, obtenu par co-réticulation d'albumine végétale extraite de légumineuses avec un ester réactif de polyéthylèneglycol. Ces hydrogels adsorbent ou mettent en liberté aisément les produits nutritifs ou des facteurs de croissance nécessaires aux cellules et/ou greffons pendant leur transport.

Le milieu de culture peut être également, en tant que polymère naturel ou semi synthétique, un gel d'acide hyaluronique non réticulé ou réticulé, thermosensible ou pH-sensible. Le gel est liquide au-dessus d'une certaine température et il est essentiellement solide en dessous d'une seconde température. La réticulation de l'acide peut être effectuée par greffage de divinylsulfone ou d'éthers glycidiques. De tels gels sont décrits dans le brevet US 5 559 880 au nom de Viratest International Inc.

US 5 040 677 décrit un système pour le transport des cellules des Kératinocytes dans un milieu liquide.

Un autre type de milieu de culture est constitué par un hydrogel mou à base de copolymère synthétique triséquencé retenant une quantité d'eau au moins égale à celle dudit copolymère. Un tel hydrogel est décrit dans la demande de brevet international WO 97/19973 au nom du CNRS.

Par ailleurs, le milieu de transport semi-solide est constitué d'un gel d'agarose à 1 à 5 % et de préférence à 1 à 2 %, ajouté au milieu de culture pour réaliser une gélification optimale.

On peut également utiliser du collagène ou de la gélatine qui forme des gels compacts.

Au moment de placer le greffon ou le fragment de tissu, il faut procéder à l'ouverture ménagée de la grande barquette contenant la petite barquette et le milieu semi-solide.

On met en position le greffon ou le fragment de tissu contre ce milieu semi-solide, on prend la petite barquette avec le greffon et on place l'ensemble dans l'empreinte de scellement.

A cause de la fragilité de l'hydrogel, une de ses faces, de préférence la face inférieure, devra en cas de besoin être renforcée par un support (backing) activé par plasma à basse pression. L'hydrogel est mis en position au centre du support afin que l'opérateur puisse au moment voulu s'en saisir avec des pinces stériles sans toucher et surtout sans risquer d'endommager l'hydrogel lui-même.

De préférence, le support sera formé d'une couche de polyéthylène de 0,05 à 1,5 mm d'épaisseur. Ses dimensions varient de 100 à 150 mm de long et de 100 à 150 mm de large. Ses dimensions et notamment son épaisseur dépendent des caractéristiques physicochimiques du gel ou de l'hydrogel utilisé. Le support peut également être un film. De préférence, le support a entre 10 et 300 µm d'épaisseur et d'une manière davantage préférée de 50 à 100 µm d'épaisseur. Le support peut être apposé ou fixé au milieu de transport, de manière à lui conférer une plus grande résistance mécanique.

Les hydrogels après préparation sont stérilisés dans un emballage spécifique au moyen de rayonnement pénétrant. Les hydrogels sont préparés au départ d'une matière première déshydratée au fond d'une barquette thermoformée en PETG, de 0,25 à 0,50 mm d'épaisseur, assez profonde, pour permettre de recevoir l'hydrogel pendant le transport et pour sa réhydratation. A cette fin, la matière première de l'hydrogel est mise en position au fond de la barquette contre les cannelures mâles pour éviter toute forme d'adhérence excessive. Le support au contraire fera face à l'utilisateur.

L'emballage des hydrogels sera constitué de la barquette thermoformée en PETG et d'un sachet étanche (aluminium, feuille de plastique interne, dans lequel on dispose l'ensemble mentionné ci-dessus).

Pour l'utilisation, le sachet contenant l'hydrogel déshydraté est ouvert pour y ajouter le milieu de réhydratation, en pratique, un milieu de culture, plus précisément le milieu de culture approprié aux cellules à transporter, puis il est scellé à nouveau.
On ouvre le sachet à nouveau lorsque l'hydrogel est réhydraté de manière à le placer dans la barquette de transport des greffons.
Il est également possible d'utiliser des hydrogels ayant une capacité de réhydratation rapide, qui sont fixés chimiquement sur ou à un support. Ce type de milieu semi-solide stérile est placé directement sous forme déshydratée dans la barquette de transport de greffons, dans laquelle s'effectue la phase de réhydratation rapide.
Pour l'emballage final des greffons au sein des barquettes, on procède comme suit : lorsque l'opérateur s'apprête à sceller une première petite barquette, la grande empreinte est laissée vide ; ceci pourrait nuire au bon fonctionnement de l'appareil. L'opérateur place alors dans la grande empreinte une grande barquette vide déjà operculée.
L'opérateur pousse ensuite la nacelle, s'assure que la température correcte de scellage a été atteinte et appuie sur les boutons de départ du cycle automatique. Il maintient sous pression les boutons jusqu'à ce que le contact soit établi entre le bloc de soudure et les barquettes. Lorsque le premier plateau supérieur est scellé, l'opérateur retire le plateau inférieur (grande barquette), vide, déjà operculé et transfère à l'aide de pinces stériles un nouveau plateau inférieur dans la grande empreinte.

L'opérateur peut ainsi positionner le plateau supérieur operculé contenant le greffon dans le plateau inférieur qui lui est assujetti et placer une portion de film pelable stérile et découpé aux dimensions appropriées.

Parallèlement, l'opérateur va répéter les opérations de chargement de la prochaine petite barquette, les opérations de positionnement du greffon dans celle-ci suivi du placement du film pelable comme cela a été décrit ci-dessus.

L'opérateur pousse à nouveau la nacelle afin d'operculer les barquettes.

L'opérateur retire le plateau inférieur operculé auquel est assujetti le plateau supérieur. Le greffon se trouve ainsi dans un double emballage stérile. Les barquettes, selon l'invention, sont ainsi prêtes pour être adressées aux établissements chirurgicaux qui pratiqueront l'implantation du greffon.

Pour l'emballage du dernier greffon, lorsque l'opérateur positionne le grand film pelable stérile sur le plateau inférieur, il place également dans la petite empreinte un plateau supérieur vide déjà operculé.

L'opérateur pousse ensuite la nacelle, s'assure que la température correcte de scellage a été atteinte et appuie sur les boutons de départ du cycle automatique. Il maintient sous pression les boutons jusqu'à ce que le contact soit établi entre le bloc de soudure et les barquettes.

La dernière barquette selon l'invention est ainsi prête pour être adressée aux établissements chirurgicaux qui pratiqueront l'implantation du greffon.
Parallèlement à la mise en place des greffons dans la barquette, l'opérateur met en route, de préférence la veille de l'emballage des greffons, le poste de sécurité microbiologique (PSM) sous la forme d'une hotte à flux laminaire, la vitre avant étant ouverte et le capot technique étant également ouvert. L'ensemble des surfaces accessibles de ce poste est désinfecté par pulvérisation humide, puis soigneusement essuyé. Le capot technique est refermé et la vitre avant est abaissée jusqu'à la position de fonctionnement normal de ce poste. Le poste de sécurité microbiologique reste en fonctionnement.

L'outillage et le matériel de transfert sont placés dans le poste de sécurité microbiologique à droite de la scelleuse.

Les emballages coulissants du type boîtage emboîtable, sont définis par le fait qu'ils sont constitués d'un plateau inférieur de forme quadrangulaire portant un ou plusieurs rebords intérieurs dont l'un présente au centre d'un côté, une solution de continuité dégageant un évidement central et d'un plateau supérieur portant un évidement intérieur et comportant une série de rebords venant s'ajuster sur le rebord central du plateau inférieur et reposant sur ledit évidement central du plateau inférieur.

Par ailleurs, les deux plateaux s'emboîtent l'un dans l'autre pour maintenir stérile le plateau supérieur et réaliser l'obturation, et ils reçoivent chacun des bandes de film stérile pour operculer les barquettes et fixer ainsi le positionnement du plateau inférieur d'une manière momentanément inamovible.

En pratique, le plateau supérieur repose par sa face externe sur le rebord inférieur du plateau inférieur et l'évidement du plateau supérieur vient s'insérer par une empreinte en creux sur le rebord en forme de bossage tourné vers la face supérieure du plateau inférieur. Ainsi, le plateau supérieur est-il maintenu en position par l'encastrement du rebord de l'évidement central contre le rebord de l'évidement symétrique du plateau inférieur.
En outre, le plateau supérieur ainsi que le plateau inférieur portent à la périphérie, de part et d'autre du plateau inférieur, des cannelures permettant d'assurer une plus grande rigidité des boîtages après assemblage. Le rebord de l'évidement central du plateau supérieur comporte, en supplément, plusieurs séries de cannelures disposées symétriquement. Ces cannelures servent à assurer le positionnement du plateau supérieur à la face inférieure de l'encastrement central de la face inférieure. L'évidement latéral du plateau inférieur joue un rôle important en permettant un coulissement facile du plateau supérieur, soit en ayant un espace libre suffisant pour passer un doigt au moins, en vue d'une extraction, soit en plaçant de part et d'autre, un ou des rubans stériles pour operculer les barquettes.

Les dimensions des barquettes selon l'invention peuvent varier dans de larges proportions en fonction des besoins médicaux et en fonction des caractéristiques de la machine de thermoformage.

D'une manière préférée, les dimensions du plateau inférieur s'échelonnent de 125 à 200 mm de long et de 100 à 120 mm de large. Dans un mode d'exécution préféré, les barquettes sont de forme carrée et les plateaux inférieurs possèdent une longueur de côté allant de 135 à 175 mm. Elles peuvent aussi être de forme rectangulaire, et posséder une longueur allant de 150 mm à 170 mm et une largeur allant de 140 à 160 mm.

Le plateau supérieur présente une longueur qui s'échelonne de 125 à 130 mm et la longueur de côté de l'évidement central de forme carrée est de 105 mm à 110 mm. L'encastrement latéral est d'environ 25 mm de largeur pour une longueur allant de 30 à 40 mm.

Les barquettes selon l'invention sont en matériau rigide transparent, notamment une matière plastique synthétique aisément moulable et thermodurcissable telle que le polyéthylène haute densité.

Elles peuvent être en toute autre matière synthétique présentant les mêmes caractéristiques physiques et stérilisables par un rayonnement pénétrant. On peut citer comme autre matériau constitutif un polycarbonate ou un acétate de polyvinyle de haut poids moléculaire.
Les cultures de cellules disposées sur un support biodégradable, à base d'acide hyaluronique, d'acide lactique, de collagène, de chitine, de chitosanne ou autres glycosaminoglycanes ou bien, sur un support inerte synthétique, c'est-à-dire non biodégradable, peuvent également bénéficier de ces types de milieux semi-solides pendant leur transport.

Le volume du plateau supérieur est gazé comme indiqué plus haut en insufflant du gaz carbonique. Après scellement, la teneur en CO₂ varie de 5 à 15 % et de préférence, de 5 à 10%.

Selon une modalité importante de l'invention, les blisters reçoivent une charge importante de gaz carbonique de façon à ce que les cultures de tissus soient toujours conditionnées sous une surpression de gaz, le gaz carbonique constituant un agent métabolique pour les cellules épithéliales. A cette fin, après scellement du plateau supérieur, on décolle les bords de la barquette et on introduit par un fin ajutage comme un petit tuyau stérile relié à une source de gaz carbonique stérile du CO₂ aussi longtemps que la barquette peut retenir ce gaz, on recolle ensuite à la main et rapidement le film pour que le blister reste bien collé et on procède à un nouveau scellement du plateau supérieur.

Selon une autre modalité importante de l'invention, les barquettes sont disposées dans une boîte isothermique où la température est régulée par la présence de « boules » ou nodules spécifiques en matière plastique, préalablement réfrigérés ou chauffés (selon les conditions climatiques extérieures) pour garantir au milieu une température compatible avec la mise en culture et le développement des cellules après la greffe.

Les milieux de culture énumérés précédemment et notamment les hydrogels co-réticulés conviennent particulièrement pour stimuler les cellules pendant le transport et pour favoriser la multiplication et la différenciation des kératinocytes après la greffe.

A l'arrivée des barquettes dans le service hospitalier de destination, celles-ci sont transférées au bloc opératoire.

Après ouverture de la barquette inférieure, et de la barquette supérieure en champ stérile, les feuillets d'épithélium cultivés sont directement pris par le chirurgien à l'aide de pinces stériles et conviennent pour l'application sur une plaie ou sur une brûlure.

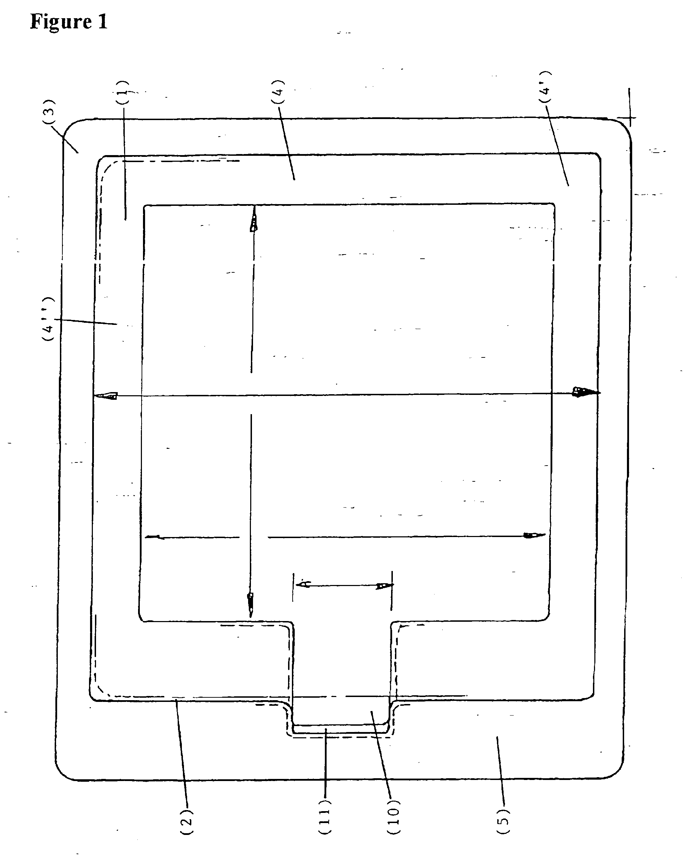

Selon une modalité particulière représentée très schématiquement à la Figure 1, on voit par au-dessus le plateau supérieur de la barquette (1) ou petite barquette reposant sur un évidement intermédiaire (2) décrivant tout le pourtour de la barquette inférieure ou grande barquette (3). La barquette supérieure de forme quadrangulaire possède trois rebords étroits (4) (4') (4'') et un rebord large (5).

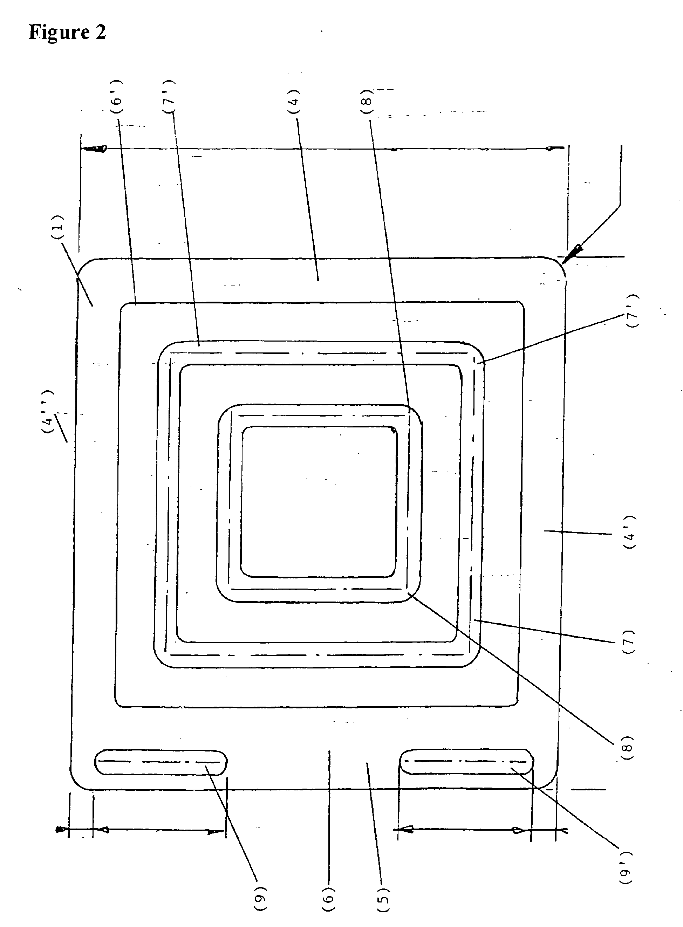

La Figure 2 montre le détail de la barquette supérieure (1) avec une partie centrale réhaussée (6) comportant deux cannelures mâles (7) (7') et (8) (8') décrivant chacune un carré. On voit figurer les trois rebords étroits (4) (4') (4") et le rebord large (5) portant, disposées symétriquement, deux cannelures mâles (9) (9').

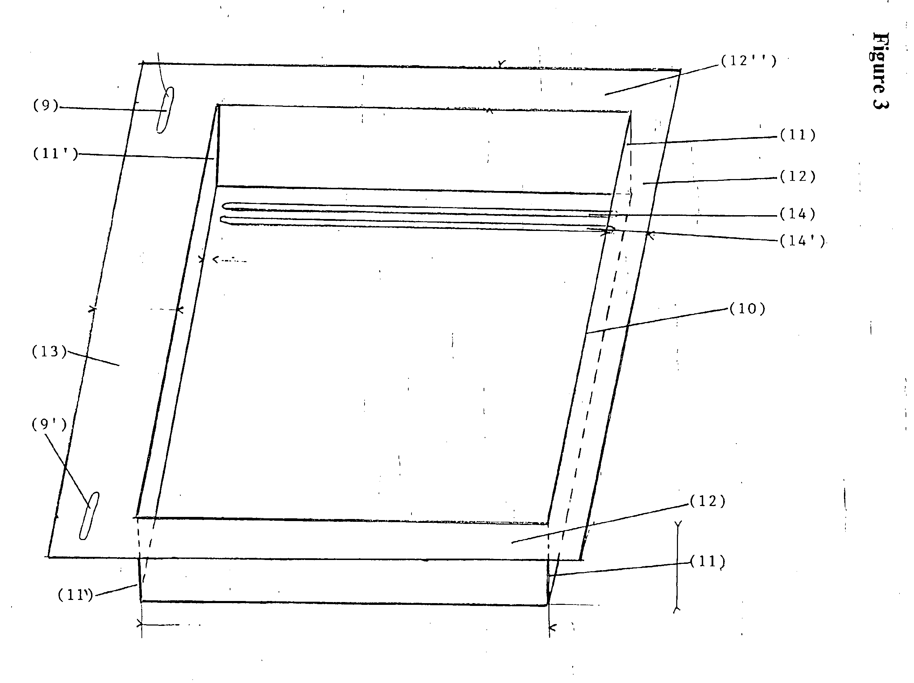

Selon une autre modalité d'exécution de l'invention représentée sur la Figure 3, la barquette de conditionnement, de transport et de réhydratation de l'hydrogel pourra présenter la forme d'un bac (10) de surface carrée ou rectangulaire à parois verticales (11) (11') présentant un rebord plus ou moins important (12) (12') (12'') et présentant sur son fond une série de cannelures mâles (14) (14'). Selon une disposition préférée, le bac (10) aura une largeur de 100 à 120 mm, une longueur de 100 à 150 mm, une hauteur de 18 à 25 mm. La largeur du rebord sera sur trois côtés (12) (12') (12") de 10 mm et sur un des côtés (13) elle sera de 20 mm. Cette grande face comporte une ou plusieurs cannelures femelles (4) (4') qui permettent le modelage du moule et de la barquette.

L'épaisseur du bac est la plus faible possible.

Les cannelures mâles présentent une surface bombée d'environ 2 mm de haut. Celles-ci permettent une meilleure réhydratation de l'hydrogel tout en évitant une adhésion potentielle du polymère au fond du bac. Le bac ainsi décrit est réalisé en une matière plastique transparente comme le polyéthylène haute densité.

C'est dans ce type de bac que l'on introduit le polymère déshydraté puis le milieu liquide comme par exemple le milieu de culture approprié pour la culture de cellules désirée. Le milieu liquide pourra être du liquide physiologique éventuellement enrichi avec certains facteurs de croissance, vitamines ou oligoéléments, ou bien un milieu de culture plus spécifique (DMEM ou F12).

Les cultures de cellules trouvent ainsi une utilisation précieuse pour la cicatrisation des plaies, notamment des plaies de grande surface, pour la réparation des tissus lésés par brûlure ou pour le comblement des cicatrices résultant d'une opération chirurgicale importante, notamment au niveau facial ou au niveau d'une plaie d'amputation, ou pour la régénération d'une plaie variqueuse ou ulcéreuse des membres inférieurs.

## Revendications

1. Procédé permettant d'assurer d'une manière optimale le transport de cellules, notamment de cellules humaines en cours de culture et notamment des greffons cutanés composés de feuillets d'épiderme, **caractérisé en ce que** :
- les feuillets d'épiderme sont placés sur un milieu semi-solide formé d'un polymère naturel, synthétique ou semi-synthétique déshydratable ou réhydratable dans un milieu de culture, aucun milieu liquide n'étant agencé pour éviter un frottement entre les cultures basales et par conséquent, pour éviter tout dommage,
- les épidermes ou épithéliums en culture sont « clipsés » à un pansement non adhérent inoffensif,
- les feuillets d'épiderme sont emballés sous flux laminaire dans un blister stérile double, étanche à l'eau et dans des conditions favorables à l'utilisateur,
- les blisters stériles intérieurs sont soumis à une injection de gaz contenant du CO₂ pour stimuler la prolifération des cellules épidermiques,
- les blisters stériles sous pression de gaz carbonique sont conditionnés à l'intérieur d'une boîte isothermique pour le transport avec des « nodules » spécifiques qui peuvent absorber et/ou engendrer de la chaleur par transport d'énergie, de façon à ce que la température pendant le transport soit ajustée à 18-28 °C.

2. Procédé selon la revendication 1 dans lequel, après transport, on effectue l'étape supplémentaire de prélèvement des cellules ou des greffons au moyen de pinces stériles.

3. Procédé selon l'une des revendications 1 ou 2 dans lequel le milieu de transport est un milieu semi-solide obtenu par co-réticulation d'albumine végétale extraite de légumineuses par réaction chimique avec un ester réactif de polyéthylèneglycol.

4. Procédé selon l'une des revendications 1 ou 2 dans lequel le milieu de transport est un milieu semi-solide formé de caséine réticulée par réaction chimique avec un ester réactif de polyéthylèneglycol.

5. Procédé selon l'une des revendications 1 ou 2 dans lequel le milieu de transport est formé d'une farine de pois condensée à un agent réticulant comme avec un ester réactif de polyéthylène glycol ou un éther bis glycidique.

6. Procédé de transport des cellules selon l'une des revendications 1 ou 2 dans lequel le milieu de culture est un gel de chitine ayant un degré d'acétylation d'au moins 60 % ou un gel de chitosane, dans l'eau.

7. Procédé de transport de cellules selon l'une des revendications 1 ou 2 dans lequel le gel de chitine est un gel contenant de 0,5 à 3,5 % de chitine.

8. Procédé de transport de cellules selon l'une des revendications-1 ou 2 dans lequel le milieu de culture est un gel d'acide hyaluronique réticulé ou non réticulé.

9. Procédé de transport de cellules selon l'une des revendications 1 ou 2 dans lequel le milieu de culture est un hydrogel mou à base de copolymère triséquencé.

10. Procédé de transport de cellules selon l'une des revendications 1 ou 2 dans lequel le milieu de transport est constitué d'un gel d'agarose ajouté au milieu de culture.

11. Procédé de transport de cellules selon l'une des revendications 1 à 10 dans lequel le transport est effectué dans des blisters doubles, étanches, et notamment des barquettes creuses thermoscellables.

12. Procédé de transport de cellules selon l'une des revendications 1 à 10 dans lequel le plateau supérieur du blister est positionné et maintenu en place à l'aide d'un film pelable, stérile et découpé aux dimensions appropriées.

13. Procédé de transport de cellules selon l'une des revendications 1 à 10 dans lequel on introduit dans les blisters une charge importante de gaz carbonique à l'aide d'un ajutage fin inséré entre les deux parois.

14. Procédé de transport de cellules selon la revendication 13 dans lequel la teneur en CO₂ dans les blisters, varie de 5 à 15 %.

15. Procédé de transport de cellules selon l'une des revendications 1 à 14 dans lequel les barquettes sont disposées dans une boîte isothermique dont la température est régulée.

16. Procédé de transport de cellules selon la revendication 15 dans laquelle la régulation de la température dans les boîtes isothermiques s'effectue à l'aide de nodules ou de boules spécifiques préalablement réfrigérés ou chauffés.

17. Utilisation des cultures de cellules transportées selon le procédé des revendications 1 à 16 pour la production d'une composition destinée à l'application sur une plaie ou sur une brûlure.

18. Utilisation des cultures de kératinocytes transportées selon le procédé des revendications 1 à 16 pour la réalisation d'une composition destinée à la reconstitution de la peau.

## Claims

1. A process for allowing the optimal transport of cells namely of human cells in culture process and mainly of cutaneous grafts composed of epidermal layers **characterised in that**:
- the epidermal layers are placed on a semi-solid medium composed of a natural, synthetic or semi-synthetic dehydratable or rehydratable polymer in a culture medium, not any liquid medium being adjusted to avoid any rubbing between the basal cultures and consequently, to avoid any harm,
- the epidermis or epithelium in culture process are clipsed to a non-adhesive harmless dressing,
- the epidermis layers are packaged under laminar flow in a watertight, steril, two-fold blister and under favourable conditions to user,
- the inner steril blisters are subjected to an injection of gas containing CO₂ to stimulate the proliferation of epidermal cells,
- the steril blisters pressurized with carbon dioxide are packaged into an isothermal box for the transport with specific "nodules" which can adsorb and/or produce heat by energy transport, so that the temperature during the transport will be adjusted from 18 to 28°C.

2. A process according to claim 1 wherein, after transport, the further step of sampling cells or grafts is performed thanks to steril clamps.

3. A process according to any of the claims 1 or 2 wherein the transport medium is a semi-solid medium obtained by co-cross-linkage of vegetable albumin extracted from leguminosae by a chemical reaction with a reactive ester of polyethylene glycol.

4. A process according to any of the claims 1 or 2 wherein the transport medium is a semi-solid medium composed of casein cross-linked by chemical reaction with a reactive ester of polyethylene glycol.

5. A process according to any of the claims 1 or 2 wherein the transport medium is made of a pea flour condensed to a cross-linking agent such as a reactive ester of polyethylene glycol or a bis-glycidic ether.

6. A process for transporting cells according to any of the claims 1 or 2 wherein the culture medium is a gel of chitin with an acetylation rate of at least 60% or a gel of chitosane, in water.

7. A process for transporting cells according to any of the claims 1 or 2 wherein the gel of chitin is a gel containing from 0.5 to 3.5% of chitin.

8. A process for transporting cells according to any of the claims 1 or 2 wherein the culture medium is a cross-linked or not cross-linked hyaluronic acid gel.

9. A process for transporting cells according to any of the claims 1 or 2 wherein the culture medium is a soft hydrogel based on triblock copolymer.

10. A process for transporting cells according to any of the claims 1 or 2 wherein the transport medium is composed of an agarose gel added to the culture medium.

11. A process for transporting cells according to any of the claims 1 to 10 wherein the transport is performed into two-fold, watertight blisters, and namely into hollow shrink-wrappable containers.

12. A process for transporting cells according to any of the claims 1 to 10 wherein the upper plate of the blister is positioned and kept in place thanks to a steril, strippable film cut to the appropriate sizes.

13. A process for transporting cells according to any of the claims 1 to 10 wherein a significant load of carbone dioxide is introduced into the blisters using a thin nozzle inserted between the two walls.

14. A process for transporting cells according to claim 13 wherein the CO₂ rate into the blisters vary from 5 to 15%.

15. process for transporting cells according to any of the claims 1 to 14 wherein the blisters are placed in an isothermal box, the temperature of which is controlled.

16. A process for transporting cells according to claim 15 wherein the temperature regulation of the isothermal box is performed by means of specific nodules or balls beforehand refrigerated or warmed up.

17. Use of the cells cultures transported according to the process of claims 1 to 16 for the production of a composition intended for application on a wound or on a burn.

18. Use of the keratinocytes cultures transported according to the process of claims 1 to 16 for the making of a composition intended for skin regeneration.

## Patentansprüche

1. Verfahren, das es ermöglicht, in optimaler Weise den Transport von Zellen, insbesondere von menschlichen Zellen in Kultur und insbesondere von Hauttransplantaten, die aus Epidermisschichten bestehen, zu gewährleisten, **dadurch gekennzeichnet, dass:**
- die Epidermisschichten auf einem halbfesten Medium angeordnet werden, das von einem dehydratisierbaren oder rehydratisierbaren natürlichen, synthetischen oder halbsynthetischen Polymer in einem Kulturmedium gebildet wird, wobei kein flüssiges Medium angeordnet wird, um eine Reibung zwischen den Basalkulturen zu vermeiden und folglich jeglichen Schaden zu vermeiden,
- die Epidermen oder Epithelien in Kultur an ein unbedenkliches nichthaftendes Verbandmaterial «geheftet» werden,
- die Epidermisschichten unter Laminar-Flow und unter für den Benutzer günstigen Bedingungen in einen wasserdichten sterilen Doppelblister gepackt werden,
- die inneren sterilen Blister einer Injektion von CO₂-haltigem Gas unterzogen werden, um die Proliferation der Keratinocyten zu stimulieren,
- die sterilen Blister unter Kohlendioxiddruck im Innern einer Isotherm-Box für den Transport mit speziellen «Kugeln» klimatisiert werden, die durch Energietransport Wärme absorbieren und/oder erzeugen können, so dass die Temperatur während des Transports auf 18-28°C eingestellt ist.

2. Verfahren nach Anspruch 1, bei dem nach dem Transport der zusätzliche Schritt der Entnahme der Zellen oder der Transplantate mittels steriler Pinzetten durchgeführt wird.

3. Verfahren nach einem der Ansprüche 1 oder 2, bei dem das Transportmedium ein halbfestes Medium ist, das durch Covernetzung von aus Leguminosen extrahiertem pflanzlichem Albumin durch chemische Reaktion mit einem reaktiven Polyethylenglycolester erhalten wird.

4. Verfahren nach einem der Ansprüche 1 oder 2, bei dem das Transportmedium ein halbfestes Medium ist, das von Kasein gebildet wird, das durch chemische Reaktion mit einem reaktiven Polyethylenglycolester vernetzt wurde.

5. Verfahren nach einem der Ansprüche 1 oder 2, bei dem das Transportmedium von einem Erbsenmehl gebildet wird, das mit einem Vernetzungsmittel wie mit einem reaktiven Polyethylenglycolester oder einem Bisglycidether kondensiert wurde.

6. Verfahren zum Transport von Zellen nach einem der Ansprüche 1 oder 2, bei dem das Kulturmedium ein Chitingel mit einem Acetylierungsgrad von wenigstens 60% oder ein Chitosangel in Wasser ist.

7. Verfahren zum Transport von Zellen nach einem der Ansprüche 1 oder 2, bei dem das Chitingel ein Gel ist, das 0,5 bis 3,5% Chitin enthält.

8. Verfahren zum Transport von Zellen nach einem der Ansprüche 1 oder 2, bei dem das Kulturmedium ein Gel aus vernetzter oder unvernetzter Hyaluronsäure ist.

9. Verfahren zum Transport von Zellen nach einem der Ansprüche 1 oder 2, bei dem das Kulturmedium ein weiches Hydrogel auf Basis eines Triblockcopolymers ist.

10. Verfahren zum Transport von Zellen nach einem der Ansprüche 1 oder 2, bei dem das Transportmedium aus einem dem Kulturmedium zugesetzten Agarosegel besteht.

11. Verfahren zum Transport von Zellen nach einem der Ansprüche 1 bis 10, bei dem der Transport in dichten Doppelblistern und insbesondere heißsiegelbaren hohlen Schalen durchgeführt wird.

12. Verfahren zum Transport von Zellen nach einem der Ansprüche 1 bis 10, bei dem die obere Schale des Blisters mittels eines sterilen abziehbaren Films, der auf die geeigneten Abmessungen zugeschnitten ist, positioniert und fixiert wird.

13. Verfahren zum Transport von Zellen nach einem der Ansprüche 1 bis 10, bei dem mittels einer dünnen Düse, die zwischen die zwei Wände eingefügt ist, eine beträchtliche Füllmenge an Kohlendioxid in die Blister eingeleitet wird.

14. Verfahren zum Transport von Zellen nach Anspruch 13, bei dem der CO₂-Gehalt in den Blistern zwischen 5 und 15% beträgt.

15. Verfahren zum Transport von Zellen nach einem der Ansprüche 1 bis 14, bei dem die Schalen in einer Isotherm-Box angeordnet werden, deren Temperatur reguliert wird.

16. Verfahren zum Transport von Zellen nach Anspruch 15, bei dem die Regulation der Temperatur in den Isotherm-Boxen mittels zuvor gekühlter oder erwärmter spezieller Knollen oder Kugeln erfolgt.

17. Verwendung der nach dem Verfahren der Ansprüche 1 bis 16 transportierten Zellkulturen für die Herstellung einer Zusammensetzung, die dazu bestimmt ist, auf eine Wunde oder Verbrennung aufgebracht zu werden.

18. Verwendung der nach dem Verfahren der Ansprüche 1 bis 16 transportierten Keratinocytenkulturen für die Herstellung einer Zusammensetzung, die zur Rekonstruktion der Haut bestimmt ist.
